# Europäisches Patentamt
## European Patent Office
## Office européen des brevets

(11) Veröffentlichungsnummer: **0 053 335**
**B1**

(12) **EUROPÄISCHE PATENTSCHRIFT**

(45) Veröffentlichungstag der Patentschrift:
16.05.84

(21) Anmeldenummer: **81109809.4**

(22) Anmeldetag: **21.11.81**

(51) Int. Cl.³: **C 07 C 19/045,** C 07 C 17/156,
C 07 C 17/02

(54) Verfahren zur Herstellung von 1,2-Dichlorethan.

(30) Priorität: **28.11.80 DE 3044854**

(43) Veröffentlichungstag der Anmeldung:
**09.06.82 Patentblatt 82/23**

(45) Bekanntmachung des Hinweises auf die Patenterteilung:
**16.05.84 Patentblatt 84/20**

(84) Benannte Vertragsstaaten:
**BE DE FR GB IT NL SE**

(56) Entgegenhaltungen:
**EP - A - 0 019 863**
**DE - A - 1 768 453**
**DE - A - 2 733 502**
**DE - B - 1 793 051**
**DE - B - 2 400 417**

(73) Patentinhaber: **HOECHST AKTIENGESELLSCHAFT,
Postfach 80 03 20, D-6230 Frankfurt am Main 80 (DE)**

(72) Erfinder: **Riedl, Josef, Dr., Kantstrasse 53,
D-8261 Burgkirchen/Alz (DE)**
Erfinder: **Kühn, Wenzel, Dr., Kampenwandstrasse 15,
D-8261 Burgkirchen/Alz (DE)**
Erfinder: **Schwarzmaier, Peter, Billerstrasse 10,
D-8261 Kastl (DE)**

BUNDESDRUCKEREI BERLIN

**0 053 335**

## Verfahren zur Herstellung von 1,2-Dichlorethan

Die Erfindung betrifft ein Verfahren zur Herstellung von 1,2-Dichlorethan gemäß Anspruch 1.

1,2-Dichlorethan wird in großem Maß erzeugt und überwiegend zur Herstellung von Vinylchlorid durch thermische Spaltung weiterverwendet. Zur Gewinnung von 1,2-Dichlorethan dient hauptsächlich die direkte Anlagerung von Chlor an Ethylen in Gegenwart katalytischer Mengen einer Lewis-Säure, meistens Eisen-III-chlorid. Diese Reaktion kann in der Gasphase oder in einer Flüssigkeit, die in der Regel überwiegende Mengen 1,2-Dichlorethan enthält, durchgeführt werden, wobei im allgemeinen der Flüssigphase-Reaktion der Vorzug gegeben wird.

Da bei der thermischen Spaltung von 1,2 Dichlorethan zu Vinylchlorid sich Chlorwasserstoff bildet, wird zu dessen Wiederverwendung meistens neben der direkten Chlorierung von Ethylen auch die Umsetzung von Ethylen mit Sauerstoff oder Luft und Chlorwasserstoff in Gegenwart eines meist kupferhaltigen Katalysators, nachfolgend als »Oxychlorierung des Ethylens« bezeichnet, durchgeführt. Eine moderne Herstellungsanlage für 1,2-Dichlorethan, das zu Vinylchlorid thermisch gespalten werden soll, enthält also vorteilhaft mindestens einen Reaktor, in dem die Direktchlorierung des Ethylens und mindestens einen Reaktor, in dem die Oxychlorierung von Ethylen durchgeführt wird. Aus beiden Reaktoren fallen nach Abtrennung des 1,2-Dichlorethans Abgase an, die umweltschädliche Stoffe enthalten und erst nach Entfernung von diesen in die Atmosphäre abgegeben werden dürfen.

Die vorliegende Erfindung bezweckt eine wirtschaftliche und umweltfreundliche Verwendung der Abgase aus der Direktchlorierung. Hier ist es hauptsächlich aus der älteren Literatur bekannt, die Abgase vom Direktchlorierungsreaktor in hintereinander geschalteten Wärmeaustauschern auf ungefähr —20°C zu kühlen, um 1,2-Dichlorethan zu kondensieren. Die nicht-kondensierten Gase werden dann in Wäschern zuerst mit Wasser und dann mit Alkali behandelt, um kleine Beträge nichtreagierten Chlors zu entfernen. Das übrig bleibende Gasgemisch kann dann beispielsweise als Brennstoff verwendet werden (siehe Chemical Engineering 27. März 1967, Seite 28, rechte Spalte, dritter Absatz). Dieses Verfahren ist störanfällig, da die Gase, die aus dem Direktchlorierungsreaktor abgezogen werden, auch geringe Mengen Wasserdampf enthalten, der bei Abkühlung auf —20°C in fester Form kondensiert und die Wärmeaustauscher verstopft. Häufigere Abstellungen zur Beseitigung der Verstopfung sind erforderlich. Hierbei wird 1,2-dichlorethanhaltiges Wasser erhalten, das erst gereinigt werden muß, bevor es ins Abwasser gegeben wird. Diese Schwierigkeit läßt sich zwar beseitigen, wenn man die Gase aus dem Direktchlorierungsreaktor nur auf etwa +1 bis +2°C abkühlt. Hierbei wird jedoch das 1,2-Dichlorethan nicht so vollständig kondensiert, es tritt eine Ausbeuteverschlechterung ein. Das Abgas aus der Kondensation enthält merkliche Mengen 1,2-Dichlorethan, das aus Gründen des Umweltschutzes nicht unbedenklich in die Atmosphäre abgegeben werden kann und also einen zusätzlichen Reinigungsaufwand, beispielsweise Aktivkohle-Absorber, erfordert. Auch die weitere Reinigung des Abgases in Wäschern ist aufwendig.

In neuerer Zeit ist man daher dazu übergegangen, das Abgas aus den Direktchlorierungsreaktoren, das einen Ethylenüberschuß enthält, zunächst von einem Großteil des partialdruckmäßig mitgeführten 1,2-Dichlorethans durch Auskondensieren mit Kältesole zu befreien, dann das Restgas auf den Eingangsdruck des Frischethylens zu komprimieren und in den Direktchlorierungsreaktor zurückzuführen.

Anstelle des Ethylenkreislaufes kann auch ein Nachreaktor verwendet werden, in welchem Dichlorethan über Füllkörper in Umlauf gehalten wird. An geeigneter Stelle wird in die Flüssigkeit eine kleine Menge Chlor eingeführt. Das freie Chlor reagiert mit dem Restethylen zu 1,2-Dichlorethan. Der Flüssigkeitszuwachs wird mit der Produktion vereinigt. Die verbliebenen Inertgase werden über eine Reinigungsanlage (Absorptions- oder Waschanlage) an die Atmosphäre abgegeben (siehe Ullmanns Enzyklopädie der technischen Chemie, Band 9 (1975) Seite 427, rechte Spalte, Absätze 2 und 3). Insbesondere das Verfahren unter Verwendung eines Nachreaktors ist aufwendig, es wird aber angewendet, da auch die Abgasrückführung in den Direktchlorierungsreaktor nicht befriedigt. Bei dieser reichern sich Nebenprodukte der Chlorierungsreaktion, die sich im Abgas befinden, beispielsweise Ethylchlorid, Vinylchlorid, Chlorwasserstoff, Kohlendioxid, Kohlenmonoxid und niedere gesättigte Kohlenwasserstoffe im System an, wodurch Chlorverlust entsteht und durch die Reaktion mit Chlor weitere unerwünschte Nebenprodukte gebildet werden.

Es wurde nun ein Verfahren gefunden, das eine wenig aufwendige Beseitigung der Abgase aus der Direktchlorierung des Ethylens ermöglicht, wobei vorteilhaft auch noch andere Abgase, die aus der Reindarstellung und Lagerung des 1,2-Dichlorethans stammen, mit einbezogen werden können.

Es handelt sich um ein Verfahren zur Herstellung von 1,2-Dichlorethan, wobei in mindestens einem Reaktionsraum Ethylen mit Chlor, gegebenenfalls in Gegenwart eines Katalysators, bei 20 bis 150°C und in mindestens einem weiteren Reaktionsraum Ethylen mit Chlorwasserstoff und Sauerstoff in Gegenwart eines Katalysators sowie gegebenenfalls unter den Reaktionsbedingungen inerter Gase, bei 180 bis 300°C umgesetzt wird, die gebildeten Reaktionsprodukte aus den Reaktionsräumen abgeführt werden und daraus die Hauptmenge des gebildeten 1,2-Dichlorethans abgeschieden wird, welches dadurch gekennzeichnet ist, daß das nach Abscheidung des 1,2-Dichlorethans aus den Reaktionsprodukten der Umsetzung des Ethylens mit Chlor übrig bleibende Gasgemisch, gegebenen-

2

falls unter Zuführung weiterer Gasströme, in den Reaktionsraum eingeführt wird, in dem Ethylen mit Chlorwasserstoff und Sauerstoff umgesetzt wird.

Die Reaktion von Ethylen mit Chlor erfolgt zweckmäßig nach bekannten Verfahren, beispielsweise in einer Flüssigkeit, die einen überwiegenden Anteil von 1,2-Dichlorethan enthält, bei Temperaturen von 20 bis 70° C in Gegenwart katalytischer Mengen, beispielsweise 0,05 bis 0,01 Gew.-%, bezogen auf die Flüssigkeit, einer oder mehrerer Lewis-Säuren, zum Beispiel Eisen-III-Chlorid. Dieses Verfahren kann auch bei höherer Temperatur, beispielsweise 83 bis 85° C, ausgeführt werden, wobei die Reaktionswärme im wesentlichen durch siedendes 1,2-Dichlorethan abgeführt wird. Es ist ferner auch möglich, die Umsetzung von Ethylen mit Chlor in der Gasphase, unter Einwirkung von ultraviolettem Licht oder von Katalysatoren, beispielsweise den obengenannten, vorzunehmen. Hierfür sind im allgemeinen Temperaturen bis herauf zu 150° C üblich.

Aus den bei der Umsetzung von Ethylen mit Chlor entstehenden Gasen wird das 1,2-Dichlorethan durch Kühlen auf Temperaturen kurz über dem Gefrierpunkt des Wassers, beispielsweise +1 oder +2° C, bei normalem Luftdruck abgeschieden. Die Anwendung tieferer Temperaturen würde zwar die Ausbeute an 1,2-Dichlorethan noch etwas erhöhen, bringt jedoch Schwierigkeiten, da die Abgase immer etwas Wasser enthalten, das bei 0° C und niedrigeren Temperaturen gefriert, wodurch die Kühlgefäße und Zuleitungen leicht verstopft werden können. Eine Trocknung der Abgase vor der Kondensation des 1,2-Dichlorethans wäre umständlich und kostspielig. Bei Anwendung des erfindungsgemäßen Verfahrens entsteht durch die Abkühlung der Abgase aus der Direktchlorierung von Ethylen auf nur etwa +1 bis +2° C kein Nachteil, da die im Abgas verbleibenden Anteile an 1,2-Dichlorethan den Oxychlorierungsreaktor offensichtlich unverändert durchlaufen und so die Ausbeute der Oxychlorierung an 1,2-Dichlorethan entsprechend erhöhen.

Die Umsetzung des Ethylens mit Chlorwasserstoff und Sauerstoff (Oxychlorierung) erfolgt ebenfalls zweckmäßig nach bekannten Verfahren bei 180 bis 300° C, vorzugsweise 200 bis 250° C, in Gegenwart eines kupferhaltigen Katalysators, der außerdem noch Alkalimetallchloride und weitere Zusatzstoffe enthalten kann. Das Kupfer, meist in Gestalt eines Kupfersalzes sowie gegebenenfalls die Alkalimetallchloride und weiteren Zusatzstoffe sind zweckmäßig an einem inerten, feinteiligen Material, beispielsweise Aluminiumoxid, adsorbiert. Die Katalysatorteilchen können im Reaktionsraum aufgeschichtet als sogenanntes Festbett oder in aufgewirbelter Form vorliegen. Anstelle von Sauerstoff wird zur Verbesserung der Wirtschaftlichkeit im allgemeinen Luft eingesetzt. Dies hat insbesondere beim Wirbelbettverfahren den weiteren Vorteil, Stickstoff als zusätzliches Inertgas zur Aufwirbelung der Katalysatorteilchen zur Verfügung zu haben.

Die nach der Umsetzung von Ethylen mit Chlorwasserstoff und Sauerstoff beziehungsweise Luft aus dem Reaktionsraum entweichenden Gase werden gekühlt, beispielsweise durch Einsprühen von alkalihaltigem Wasser. Hierdurch wird restlicher Chlorwasserstoff aus den Gasen entfernt. Zweckmäßig stellt man die Kühlung so ein, daß das bei Normaldruck ausgetragene, gekühlte Gas eine Temperatur oberhalb des Siedepunktes von 1,2-Dichlorethan (ca. 83° C) aufweist. Aus dem zur Kühlung verwendeten Wasser wird gelöstes 1,2-Dichlorethan durch Abstreifen mit Wasserdampf entfernt, kondensiert, vom mitkondensierten Wasserdampf getrennt und mit der Hauptmenge des gebildeten flüssigen 1,2-Dichlorethans zunächst in einem Behälter gesammelt und von diesem in eine Destillationskolonne zur Entwässerung von 1,2-Dichlorethan (sogenannte »Entwässerungskolonne«) gegeben.

Die auf beispielsweise ca. 90° C abgekühlte Hauptmenge der Gase aus der Oxychlorierung wird nun beispielsweise vermittels eines Solekühlers auf eine Temperatur abgekühlt, die etwas über dem Gefrierpunkt des Wassers liegt. Hierbei kondensiert die Hauptmenge des 1,2-Dichlorethans und wird in einen Sammelbehälter abgeleitet. Die nichtkondensierten Anteile werden nun zweckmäßig durch eine Absorptionsanlage geleitet, um den größten Teil des tensionsmäßig in den Gasen noch vorhandenen 1,2-Dichlorethans aus dem Abgas zu entfernen. Hierzu können beispielsweise Absorber aus Aktivkohle dienen oder hochsiedende, flüssige, alkylierte, aromatische Kohlenwasserstoffe, wie sie beispielsweise unter dem Handelsnamen »Solvesso«® von Firma Esso Chemie oder »Shellsol«® von Firma Shell bekannt sind. Das Abgas, welches den oder die Absorber verläßt, kann nun entweder in den Oxychlorierungsreaktor wieder eingespeist werden, oder einer nachträglichen Chlorierung mit anschließender Abtrennung des gebildeten 1,2-Dichlorethans unterzogen werden oder in einer Verbrennung miteingespeist oder als solches in die Atmosphäre abgegeben werden.

Das in den Absorptionsanlagen sich anreichernde 1,2-Dichlorethan wird von Zeit zu Zeit durch Erwärmen des Absorbens, gegebenenfalls unter Unterdruck, desorbiert, kondensiert und ebenfalls in den Sammeltank für 1,2-Dichlorethan gegeben.

Erfindungsgemäß wird das Abgas aus der Direktchlorierung des Ethylens, welches nach Kühlung auf eine Temperatur kurz über dem Gefrierpunkt des Wassers, Kondensation und Abscheidung der Hauptmenge des 1,2-Dichlorethans verbleibt, in den Reaktionsraum eingespeist, in dem Ethylen mit Chlorwasserstoff und Sauerstoff beziehungsweise Luft umgesetzt wird. Die Einspeisung kann zusammen mit dem Ethylen und/oder dem Chlorwasserstoff erfolgen, die Abgase aus der Direktchlorierung können jedoch auch an anderer Stelle in den Oxychlorierungsreaktor gegeben werden. In jedem Fall soll die Einspeisung an einer Stelle erfolgen, von der aus das Abgas aus der Direktchlorierung noch den ganzen oder einen großen Teil des Raumes durchströmt, der die Katalysatorteilchen enthält.

Vor Einspeisung in den Oxychlorierungsreaktor müssen die Abgase aus der Direktchlorierung auf

3

einen Druck gebracht werden, der etwas über dem Druck liegt, der im Oxychlorierungsreaktor an der Einspeisungsstelle der Abgase herrscht. Hierfür wird gegebenenfalls ein Kompressor verwendet.

Den Restgasen, die aus der Umsetzung von Ethylen mit Chlor nach Abtrennung der Hauptmenge des 1,2-Dichlorethans verbleiben, werden vorteilhaft noch Abgase beigemischt, die aus Destillationsapparaten zur Reinigung von 1,2-Dichlorethan und/oder aus Lagergefäßen für 1,2-Dichlorethan stammen, welche im Anschluß an die unvollständige thermische Spaltung von 1,2-Dichlorethan zur Gewinnung von Vinylchlorid nach Abtrennung des Vinylchlorids benutzt werden. Solche Lagergefäße sind beispielsweise ein Gefäß, in dem das feuchte 1,2-Dichlorethan, wie es von der Direktchlorierung und Oxychlorierung kommt, gesammelt wird, ein Gefäß, in dem das entwässerte 1,2-Dichlorethan gelagert wird und ein Gefäß, in dem das aus der Direktchlorierung von Ethylen in Gegenwart von Lewis-Säuren, beispielsweise Eisen-III-chlorid, hergestellte 1,2-Dichlorethan mit Wasser gewaschen wird, um Katalysatorreste zu entfernen.

Abgase, die aus Destillationsapparaten zur Reinigung von 1,2-Dichlorethan stammen sind solche, die nach Kondensation der Hauptmenge der kondensierbaren Anteile aus den Kopfprodukten der jeweiligen Kolonnen gasförmig verbleiben. Destillationsapparate zur Reinigung von 1,2-Dichlorethan sind zum Beispiel der Apparat, in dem 1,2-Dichlorethan von Wasser und Leichtsiedern befreit wird (Entwässerungskolonne), der Apparat, in dem 1,2 Dichlorethan von den Hochsiedern abgetrennt wird (Hochsiederkolonne) und der Apparat, in dem aus dem Sumpfprodukt der Hochsiederkolonne darin noch enthaltenes 1,2-Dichlorethan unter Verwendung von Unterdruck abdestilliert wird (Vakuumkolonne). Alle diese Abgase enthalten neben Stickstoff tensionsmäßige Mengen gasförmiges 1,2-Dichlorethan. Ein Gehalt an weiteren gasförmigen Stoffen, beispielsweise Chlorwasserstoff und Ethylen stört nicht. Die Zumischung aller dieser Abgase zum Restgas aus der Direktchlorierung erfolgt zweckmäßig vor der Komprimierung und gegebenenfalls Erwärmung zwecks Einspeisung in den Oxychlorierungsreaktor.

Nach der Kompression des Restgases aus der Direktchlorierung, gegebenenfalls in Mischung mit den Abgasen aus den in den vorhergehenden Abschnitten beschriebenen Apparaten, werden die Abgase vorteilhaft vor Eingabe in den Oxychlorierungsreaktor auf 100 bis 200°C, insbesondere auf 150 bis 180°C, erwärmt. Hierzu werden beispielsweise übliche Wärmeaustauscher verwendet, als Heizmittel kann vorteilhaft das aus dem Oxychlorierungsreaktor ausgetragene Gas verwendet werden.

In einer weiteren bevorzugten Ausführungsform des erfindungsgemäßen Verfahrens wird das Gasgemisch, welches aus der Umsetzung von Ethylen mit Chlor stammt und dem gegebenenfalls weitere Abgase zugemischt werden, auf seinen Gehalt an Chlorwasserstoff und Ethylen analysiert, beispielsweise durch gaschromatographische Analyse und das Analysenergebnis zur automatischen Steuerung der Einspeisung zusätzlicher Mengen von mindestens einem der Gase Chlorwasserstoff, Luft oder Ethylen in den Reaktionsraum benutzt wird, in dem Ethylen mit Chlorwasserstoff und Sauerstoff umgesetzt werden. In dem zuletzt genannten Oxychlorierungsreaktor reagiert 1 Mol Ethylen mit 2 Mol Chlorwasserstoff und einem halben Mol Sauerstoff (beziehungsweise fünf halbe Mol Luft) unter Bildung von 1 Mol 1,2-Dichlorethan und einem Mol Wasser. Liegt in dem Abgas, beziehungsweise Abgasgemisch, das in den Oxychlorierungsreaktor eingespeist wird, das Molverhältnis von Chlorwasserstoff zu Ethylen unter 2, so wird zweckmäßig eine zusätzliche Menge Chlorwasserstoff, liegt es über 2, so wird zweckmäßig eine zusätzliche Menge Ethylen in den Oxychlorierungsreaktor eingegeben, so daß unter Berücksichtigung aller Gase, die in den Oxychlorierungsreaktor eingespeist werden, ein Molverhältnis von Chlorwasserstoff zu Ethylen von etwa 2 gegeben ist. Im allgemeinen wird mit einem geringen molaren Überschuß von Sauerstoff beziehungsweise Luft über die weiter oben angegebenen stöchiometrischen Molarmengen hinaus gearbeitet.

Das neue Verfahren eignet sich besonders für Restgase, die aus einer Umsetzung von Ethylen mit Chlor stammen, welche in Gegenwart von flüssigem 1,2-Dichlorethan und einem Katalysator bei 20 bis 95°C durchgeführt worden ist.

Das erfindungsgemäße Verfahren ermöglicht eine kostengünstige Verwendung der Abgase aus der direkten Chlorierung des Ethylens und aus Apparaten und Lagergefäßen, die bei der Reindarstellung des 1,2-Dichlorethans benutzt werden. Es wird außerdem eine Ausbeuteverbesserung an 1,2-Dichlorethan erzielt. Ein weiterer Vorteil ist die Tatsache, daß die Abgase aus der Umsetzung von Ethylen mit Chlor nicht unter 0°C gekühlt zu werden brauchen, um eine möglichst vollständige Ausbeute an 1,2-Dichlorethan bei gleichzeitiger Reinigung der Abgase zu erzielen, wobei Verstopfungen der Kondensiereinheiten durch gefrierendes Wasser vermieden wird. Außerdem sind durch das neue Verfahren Schwankungen in den Betriebsbedingungen der direkten Chlorierung von Ethylen, beispielsweise Schwankungen der Katalysatorkonzentration oder Schwankungen in der Zuführung der Rohstoffe sowie der Betriebstemperatur wesentlich weniger wirksam auf Qualität und Ausbeute des erzeugten 1,2-Dichlorethans.

Beispiel 1

Ein kühlbarer Reaktor enthält eine Flüssigkeit, die zum größten Teil aus 1,2-Dichlorethan besteht. Im Restanteil ist hauptsächlich 1,2-Trichlorethan neben anderen chlorierten Kohlenwasserstoffen mit 1 und 2 C-Atomen vorhanden. Die Flüssigkeit enthält außerdem 0,02 bis 0,04 Gew.-% Eisen-III-chlorid (die Konzentration schwankt während des Betriebes innerhalb der angegebenen Grenzen). In den Reaktor werden Ethylen und Chlor gasförmig im Molverhältnis von 1 : 1,02 eingespeist und die Flüssigkeit umgepumpt. Die Temperatur der Flüssigkeit wird durch Kühlung auf 45°C eingestellt und gehalten. Der Druck im Reaktor beträgt 110 kPa.

Durch Abführen eines Teils der Flüssigkeit wird die Standhöhe der Flüssigkeit im Reaktor gehalten. Die abgeführte Flüssigkeit wird in einem üblichen Waschgefäß mit Wasser gewaschen, vom Waschwasser getrennt und in einem ersten Sammelbehälter gelagert. Das aus dem Reaktor entweichende Gasgemisch wird in einem Solekühler auf +3°C abgekühlt. Die hierbei kondensierte Flüssigkeit wird ebenfalls in den ersten Sammelbehälter gegeben. Aus dem Gasgemisch, das den Solekühler verläßt, werden 300 Gewichtsteile je Stunde entnommen und wie auf Seite 13, Zeilen 12 bis 14, beschrieben weiterverarbeitet.

Die Flüssigkeit aus dem ersten Sammelgefäß wird einer Destillationsapparatur zugeleitet, in der nach bekannten Verfahren Wasser und Stoffe, die bei niedrigerer Temperatur als 1,2-Dichlorethan sieden, zusammen mit etwas 1,2-Dichlorethan über Kopf abdestilliert und kondensiert werden. Das Kondensat wird in einen zweiten Sammelbehälter geleitet und anschließend in üblicher Weise aufgearbeitet, um weiteres 1,2-Dichlorethan zu gewinnen. Das im Sumpf der Kolonne verbleibende Produkt wird ebenfalls nach bekannten Verfahren zu reinem 1,2-Dichlorethan weiterverarbeitet und dieses dann einer Anlage zwecks thermischer Spaltung zu Vinylchlorid zugeführt.

Die Wäsche des Roh-1,2-Dichlorethans aus dem Reaktor, in dem Ethylen mit Chlor umgesetzt wird, die Sammlung der Flüssigkeit im ersten und zweiten Sammelbehälter wie auch die Destillation zur Abtrennung von Wasser und Leichtsiedern von 1,2-Dichlorethan werden unter Stickstoff als Schutzgas durchgeführt.

Aus dem nicht-kondensierten Abgas der oben beschriebenen Destillationsapparatur werden 530 Gewichtsteile je Stunde entnommen. Aus der jeweiligen Beatmung des 1,2-Dichlorethan-Wäschebehälters sowie des ersten und zweiten Sammelbehälters werden zusammen 20,1 Gewichtsteile je Stunde Abgas entnommen. Alle Abgas-Entnahmen werden vereinigt. Die so erhaltenen 850,1 (300 + 530 + 20,1) Gew.-Teile/h Abgas werden gaschromatographisch analysiert. Hierbei werden folgende Werte ermittelt.

| | |
|---|---|
| Wasserstoff | 5,2 |
| Sauerstoff | 14,7 |
| Stickstoff | 336,6 |
| Kohlenmonoxid | 5,0 |
| Kohlendioxid | 281,9 |
| Ethan | 6,0 |
| Ethylen | 38,0 |
| Vinylchlorid | 1,5 |
| 1,2-Dichlorethan | 34,5 |
| Methylchlorid | 2,6 |
| 1,1-Dichlorethan | 1,4 |
| Ethylchlorid | 106,1 |
| 1,1-Dichlorethylen | 0,3 |
| 1,2-Dichlorethylen-trans | 0,3 |
| 1,2-Dichlorethylen-cis | 0,3 |
| Tetrachlorkohlenstoff | 3,8 |
| Chloroform | 11,9 |
| | 850,1 |

Dieses Abgasgemisch wird auf 360 kPa Druck komprimiert, in einem Wärmetauscher auf 175°C erwärmt und einem Reaktor zugeführt, in dem Ethylen, Chlorwasserstoff und Luft in Gegenwart aufgewirbelter Katalysatorteilchen umgesetzt werden. Die Katalysatorteilchen bestehen aus Aluminiumoxid, das Kupfer-II-Chlorid und Kaliumchlorid absorbiert enthält. Das Abgasgemisch wird unterhalb der Zone, in der sich die aufgewirbelten Katalysatorteilchen befinden, in den Reaktor eingeleitet.

Aufgrund der oben angegebenen Analyse werden zur Überführung des im Abgas vorhandenen Ethylens und Ethylenchlorids in 1,2-Dichlorethan 166,4 Gew.-Teile/h Luft und 164 Gew.-Teile/h Chlorwasserstoff benötigt.

Der Reaktor wird mit dem in nachstehender Tabelle 1 unter Beispiel 1 angegebenen Mengen Luft, Chlorwasserstoff und Ethylen beschickt. Der Druck im Reaktor beträgt 350 kPa, die Temperatur 220°C. Das den Reaktor verlassende Gasgemisch wird durch Einsprühen von alkalischem Wasser auf 89°C gekühlt, das Kühlwasser anschließend mit Wasserdampf abgestreift, das so erhaltene gasförmige Gemisch aus Wasser und rohem 1,2-Dichlorethan durch Kühlen auf 5°C kondensiert, das Roh-1,2-Dichlorethan vom Wasser getrennt und in den weiter oben beschriebenen ersten Sammelbehälter gegeben.

Das auf 89°C gekühlte Gasgemisch wird in Wasserkühlern und einem Solekühler auf 10°C gekühlt

5

und das hierdurch kondensierte Roh-1,2-Dichlorethan ebenfalls in den weiter oben beschriebenen ersten Sammelbehälter geleitet. Das nicht-kondensierte Gas wird in einem Gaswäscher mit einem Gemisch aus alkylierten Benzolen, bekannt unter dem Handelsnamen Solvesso®, in Kontakt gebracht. Das den Wäscher verlassende Gas wird gaschromatographisch analysiert. Die ermittelten Werte sind in Tabelle 2 unter Beispiel 1 angegeben. Dieses Abgas wird in die Atmosphäre entlassen.

Die mit Produkten aus dem Gasgemisch angereicherte Waschflüssigkeit wird bei Unterdruck auf über 100°C erhitzt, das entweichende Gas kondensiert und das so erhaltene Roh-1,2-Dichlorethan in den oben bereits mehrfach erwähnten ersten Sammelbehälter gegeben. Die so behandelte Waschflüssigkeit geht zurück in den Gaswäscher.

Neben den in den vorangegangenen Abschnitten bereits erwähnten Werten sind in Tabelle 1 unter Beispiel 1 noch folgende Werte aufgeführt: Die Gesamtmenge des im Oxychlorierungs-Reaktor erzeugten Roh-1,2-Dichlorethans, dessen Gehalt an reinem 1,2-Dichlorethan (gaschromatographisch ermittelt; siehe Tabelle 3), die daraus errechnete Menge an reinem 1,2-Dichlorethan, die Zunahme dieser Menge gegenüber dem nachfolgend beschriebenen Vergleichsversuch A und die prozentualen Umsätze des Chlorwasserstoffs und Ethylens.

In nachfolgender Tabelle 3 ist unter Beispiel 1 die gaschromatographische Analyse des Roh-1,2-Dichlorethans aufgeführt.

## Vergleichsversuch A

Die Oxychlorierung des Ethylens wird wie vorstehend unter Beispiel 1 beschrieben durchgeführt mit einem Unterschied, daß kein Abgas in den Reaktor eingeleitet wird und die in den Tabellen 1, 2 und 3 unter Vergleich A angegebenen Verfahrensparameter angewendet beziehungsweise Werte gefunden werden.

## Beispiel 2

Es wird verfahren wie in Beispiel 1 beschrieben mit folgenden Unterschieden:

a) Die Flüssigkeit im Reaktor, in dem Ethylen mit Chlor umgesetzt wird, enthält 0,001 bis 0,003 Gew.-% Eisen-III-Chlorid, bezogen auf die Flüssigkeit (die Konzentration schwankt während des Betriebes innerhalb der angegebenen Grenzen).

b) Die Flüssigkeit im Reaktor siedet, die Temperatur beträgt 84°C.

c) Durch Abführen des gasförmigen 1,2-Dichlorethans und Kondensation unter Konstanthalten des Füllvolumens des Reaktors wird die Temperatur auf der genannten Höhe gehalten.

d) Das kondensierte und abgeführte 1,2-Dichlorethan wird nicht mit Wasser gewaschen.

e) Die unter c) beschriebene Kondensation wird im Solekühler bis zu einer Gastemperatur von +2°C fortgeführt, dem nicht kondensierten Gasgemisch werden 490 Gew.-Teile je Stunde entnommen. Die gaschromatographische Analyse dieses Gasgemisches ergibt:

|  | Gew.-Teile/h |
|---|---|
| Stickstoff | 43,8 |
| Sauerstoff | 10,0 |
| Wasserstoff | 3,1 |
| Kohlenmonoxid | 4,4 |
| Kohlendioxid | 138,4 |
| Ethan | 4,7 |
| Ethylen | 229,3 |
| Ethylenchlorid | 10,1 |
| 1,2-Dichlorethan | 46,2 |
|  | 490,0 |

Dieses Gasgemisch wird ohne Zumischung weiterer Abgase auf 360 kPa komprimiert, in einem Wärmetauscher auf 150°C erwärmt und wie in Beispiel 1 beschrieben, dem Reaktor zugeführt, in dem Ethylen, Chlorwasserstoff und Luft in Gegenwart aufgewirbelter Katalysatorteilchen umgesetzt werden.

Aufgrund der oben angegebenen Analyse werden zur Überführung des im Abgas vorhandenen Ethylens und Ethylchlorid in 1,2-Dichlorethan 665,6 Gew.-Teile/h Luft und 574 Gew.-Teile/h Chlorwasserstoff benötigt.

f) Der Oxychlorierungs-Reaktor wird bei 350 kPa und einer Temperatur von 230°C mit den in Tabelle 1 unter Beispiel 2 aufgeführten Mengen Luft, Chlorwasserstoff und Ethylen betrieben. Die Aufarbeitung der Reaktionsprodukte erfolgt wie im Beispiel 1. Die ermittelten Werte für Roh-1,2-Dichlorethan, reines 1,2-Dichlorethan und die Umsätze sind ebenfalls in Tabelle 1 unter Beispiel 2 angegeben.

Die Analyse des Roh-1,2-Dichlorethans ist in Tabelle 3, die des Abgases aus der Oxychlorierung in Tabelle 7 jeweils unter Beispiel 2 aufgeführt.

## Vergleichsversuch B

Die Oxychlorierung des Ethylens wird wie vorstehend unter Beispiel 2 beschrieben durchgeführt mit dem Unterschied, daß kein Abgas in den Reaktor eingeleitet wird und die in den Tabellen 1, 2 und 3 unter Vergleich B angegebenen Verfahrensparameter angewendet beziehungsweise Werte gefunden werden.

## Beispiel 3

Es wird verfahren wie in Beispiel 1 beschrieben mit folgenden Unterschieden:

a) Die Temperatur der Flüssigkeit im Reaktor, in dem Ethylen mit Chlor umgesetzt wird, beträgt 35°C und wird durch Kühlung auf dieser Höhe gehalten.
b) Aus dem Gasgemisch, das den Solekühler verläßt, werden 422 Gewichtsteile je Stunde entnommen. Die gaschromatographische Analyse dieses Gasgemisches ergibt:

|                     | Gew.-Teile/h |
|---------------------|--------------|
| Stickstoff          | 174,4        |
| Sauerstoff          | 11,9         |
| Wasserstoff         | 3,5          |
| Kohlenmonoxid       | 4,1          |
| Kohlendioxid        | 148,5        |
| Chlorwasserstoff    | 7,9          |
| Ethylen             | 15,0         |
| Ethylchlorid        | 8,2          |
| 1,2-Dichlorethan    | 40,9         |
|                     | 422,0        |

Dieses Gasgemisch wird ohne Zumischung weiterer Abgase und ohne Erwärmung auf 360 kPa komprimiert und, wie in Beispiel 1 beschrieben, mit einer Temperatur von 30°C dem Reaktor zugeführt, in dem Ethylen, Chlorwasserstoff und Luft in Gegenwart aufgewirbelter Katalysatorteilchen umgesetzt werden. Nach der oben angegebenen gaschromatographischen Analyse wird die Zufuhr von Chlorwasserstoff und Luft zum Oxychlorierungsreaktor automatisch gesteuert.
c) Der Oxychlorierungs-Reaktor wird bei 350 kPa und einer Temperatur von 190°C mit den in Tabelle 1 unter Beispiel 3 aufgeführten Mengen Luft, Chlorwasserstoff und Ethylen betrieben. Die Aufarbeitung der Reaktionsprodukte erfolgt wie in Beispiel 1. Die ermittelten Werte für Roh-1,2-Dichlorethan, reines 1,2-Dichlorethan und die Umsätze sind ebenfalls in Tabelle 1 unter Beispiel 3 angegeben. Die Analyse des Roh-1,2-Dichlorethans ist in Tabelle 3, die des Abgases aus der Oxychlorierung in Tabelle 2, jeweils unter Beispiel 3 aufgeführt.

## Vergleichsversuch C

Die Oxychlorierung des Ethylens wird wie vorstehend unter Beispiel 3 beschrieben durchgeführt, mit dem Unterschied, daß kein Abgas in den Reaktor eingeleitet wird und die in den Tabellen 1, 2 und 3 unter Vergleich C angegebenen Verfahrensparameter angewendet beziehungsweise Werte gefunden werden.

## Beispiel 4

Es wird verfahren wie in Beispiel 1 beschrieben, mit folgenden Unterschieden:

a) Die Flüssigkeit im Reaktor, in dem Ethylen mit Chlor umgesetzt wird, enthält 0,001 bis 0,003 Gew.-% Eisen-III-Chlorid, bezogen auf die Flüssigkeit (die Konzentration schwankt während des Betriebes innerhalb der angegebenen Grenzen).
b) Die Flüssigkeit im Reaktor siedet, die Temperatur beträgt 145°C, der Reaktor arbeitet unter Druck.
c) Durch Abführen des gasförmigen 1,2-Dichlorethans und Kondensation unter Konstanthalten des Füllvolumens des Reaktors wird die Temperatur im Reaktor auf 145°C gehalten.

d) Das kondensierte und abgeführte 1,2-Dichlorethan wird nicht mit Wasser gewaschen.

e) Die unter c) beschriebene Kondensation wird im Solekühler bis zu einer Gastemperatur von +2°C fortgeführt, das nicht-kondensierte Gasgemisch wird entspannt, mit weiteren Abgasen aus Behältern der 1,2-Dichlorethan-Lagerung und -Weiterverarbeitung gemischt und die so erhaltenen 842,5 Gewichtsteile/h gaschromatographisch analysiert. Hierbei werden folgende Werte ermittel:

|  | Gew.-%-Teile/h |
|---|---|
| Stickstoff | 70,3 |
| Sauerstoff | 13,4 |
| Wasserstoff | 3,4 |
| Kohlenmonoxid | 4,7 |
| Kohlendioxid | 117,6 |
| Ethan | 12,8 |
| Ethylen | 542,4 |
| Ethylchlorid | 9,1 |
| 1,2-Dichlorethan | 68,8 |
|  | 842,5 |

Dieses Gasgemisch wird auf 360 kPa komprimiert, in einem Wärmetauscher auf 190°C erwärmt und, wie in Beispiel 1 beschrieben, dem Reaktor zugeführt, in dem Ethylen, Chlorwasserstoff und Luft in Gegenwart aufgewirbelter Katalysatorteilchen umgesetzt werden. Aufgrund der oben angegebenen Analyse werden zur Überführung des im Abgas vorhandenen Ethylens und Ethylchlorids in 1,2-Dichlorethan 1216 Gewichtsteile/h Luft und 1394 Gewichtsteile/h Chlorwasserstoff benötigt.

f) Der Oxychlorierungsreaktor wird bei 350 kPa und einer Temperatur von 280°C mit den in Tabelle 1 unter Beispiel 4 aufgeführten Mengen Luft, Chlorwasserstoff und Ethylen betrieben. Die Aufarbeitung der Reaktionsprodukte erfolgt wie in Beispiel 1. Die ermittelten Werte für Roh-1,2-Dichlorethan, reines 1,2-Dichlorethan und die Umsätze sind ebenfalls in Tabelle 1 unter Beispiel 4 angegeben. Die Analyse des Roh-1,2-Dichlorethans ist in Tabelle 3, die des Abgases aus der Oxychlorierung in Tabelle 2, jeweils unter Beispiel 4 aufgeführt.

### Vergleichsversuch D

Die Oxychlorierung des Ethylens wird wie vorstehend unter Beispiel 4 beschrieben durchgeführt, mit dem Unterschied, daß kein Abgas in den Reaktor eingeleitet wird und die in den Tabellen 1, 2 und 3 unter Vergleich D angegebenen Verfahrensparameter angewendet beziehungsweise Werte gefunden werden.

Tabelle 1

Verfahrensbedingungen und ermittelte Produktwerte bei der Oxychlorierung des Ethylens

|  | Ver-gleich A | Bei-spiel 1 | Ver-gleich B | Bei-spiel 2 | Ver-gleich C | Bei-spiel 3 | Ver-gleich D | Bei-spiel 4 |
|---|---|---|---|---|---|---|---|---|
| Luft, Gew.-Teile/h | 46 920 | 47 086 | 46 920 | 47 586 | 46 850 | 46 882 | 47 042 | 48 258 |
| HCl, Gew.-Teile/h | 31 240 | 31 404 | 31 650 | 32 224 | 31 158 | 31 200 | 31 158 | 32 552 |
| $C_2H_4$, Gew.-Teile/h | 12 580 | 12 580 | 12 580 | 12 580 | 12 310 | 12 310 | 12 310 | 12 310 |
| Temperatur, °C | 220 | 220 | 230 | 230 | 190 | 190 | 280 | 280 |
| zugefahrenes Abgas, Gew.-Teile/h | — | 850,1 | — | 490 | — | 422 | — | 842,5 |

Tabelle 1 (Fortsetzung)

| | Ver-gleich A | Bei-spiel 1 | Ver-gleich B | Bei-spiel 2 | Ver-gleich C | Bei-spiel 3 | Ver-gleich D | Bei-spiel 4 |
|---|---|---|---|---|---|---|---|---|
| Roh-1,2-Dichlor-ethan, Gew.-Teile/h | 41 965 | 42 270 | 42 570 | 43 400 | 41 000 | 41 095 | 41 435 | 42 885 |
| Prozentgehalt 1,2-Dichlorethan | 99,19 | 99,065 | 98,969 | 98,960 | 99,225 | 99,201 | 97,053 | 96,954 |
| reines 1,2-Dichlorethan, Gew.-Teile/h | 41 595 | 41 870 | 42 130 | 42 950 | 40 680 | 40 765 | 40 210 | 41 575 |
| Zunahme an 1,2-Dichlorethan in Gew.-Teilen und Prozent | — = 0,66% | 275 | — = 1,95% | 820 | — = 0,2% | 85 | — = 3,4% | 1 365 |
| Umsatz HCl | 99,6% | 99,6% | 99,7% | 99,7% | 97,7% | 97,8% | 99,8% | 99,8% |
| Umsatz $C_2H_4$ zu Roh-1,2-Dichlorethan | 94,2% | [1] | 95,3% | [1] | 94,2% | [1] | 93,3% | [1] |
| zu $CO_2$ | 2,3% | | 1,95% | | 1,9% | | 5,3% | |
| zu CO | 1,2% | | 0,90% | | 0,7% | | 0,2% | |
| $C_2H_4$ unverändert im Abgas | 2,3% | | 1,85% | | 3,2% | | 1,2% | |
| | 100,00% | | 100,00% | | 100,00% | | 100,00% | |

[1] Umsatz ähnlich dem entsprechenden Vergleichsversuch A bzw. B, C oder D, Bestimmung unge-nau wegen Beimengungen aus dem zugefahrenen Abgas.

Tabelle 2

Analyse des Abgases aus der Oxychlorierung nach der Wäsche

| | Ver-gleich A Vol.-% | Bei-spiel 1 Vol.-% | Ver-gleich B Vol.-% | Bei-spiel 2 Vol.-% | Ver-gleich C Vol.-% | Bei-spiel 3 Vol.-% | Ver-gleich D Vol.-% | Bei-spiel 4 Vol.-% |
|---|---|---|---|---|---|---|---|---|
| $O_2$ | 6,3 | 6,2 | 6,5 | 6,4 | 7,3 | 7,3 | 1,2 | 1,3 |
| CO | 0,76 | 0,76 | 0,56 | 0,57 | 0,46 | 0,48 | 0,2 | 0,25 |
| $CO_2$ | 1,45 | 1,86 | 1,25 | 1,46 | 1,15 | 1,28 | 4,0 | 4,4 |
| $C_2H_4$ | 0,72 | 0,71 | 0,58 | 0,58 | 1,01 | 1,05 | 0,44 | 0,41 |
| $C_2H_6$ | <0,01 | 0,01 | <0,01 | 0,01 | <0,01 | 0,02 | <0,01 | 0,01 |
| Vinylchlorid | 0,0020 | 0,0024 | 0,0018 | 0,0020 | 0,0020 | 0,0021 | 0,0129 | 0,0099 |
| $C_2H_5Cl$ | 0,0045 | 0,0166 | 0,0040 | 0,0056 | 0,0045 | 0,0090 | 0,0081 | 0,0102 |
| 1,2-Dichlorethan | 0,0001 | 0,0002 | 0,0002 | 0,0001 | 0,0001 | 0,0001 | 0,0001 | 0,0001 |

Tabelle 3

Analyse des in der Oxychlorierung erzeugten Roh-1,2-Dichlorethans

| | Ver-gleich A Gew.-% | Bei-spiel 1 Gew.-% | Ver-gleich B Gew.-% | Bei-spiel 2 Gew.-% | Ver-gleich C Gew.-% | Bei-spiel 3 Gew.-% | Ver-gleich D Gew.-% | Bei-spiel 4 Gew.-% |
|---|---|---|---|---|---|---|---|---|
| $H_2O$ | 0,192 | 0,190 | 0,196 | 0,194 | 0,190 | 0,188 | 0,192 | 0,184 |
| Summe $C_2H_2$; $C_2H_4$; $C_2H_6$ | 0,009 | 0,009 | 0,007 | 0,007 | 0,008 | 0,009 | 0,002 | 0,002 |
| Vinylchlorid | 0,002 | 0,002 | 0,002 | 0,002 | 0,002 | 0,002 | 0,024 | 0,028 |
| $C_2H_5Cl$ | 0,008 | 0,031 | 0,007 | 0,011 | 0,008 | 0,014 | 0,098 | 0,102 |
| Ethylenoxid | 0,003 | 0,003 | 0,003 | 0,003 | 0,003 | 0,003 | 0,003 | 0,003 |
| 1,2-Dichlor-ethylen-trans | 0,002 | 0,002 | 0,002 | 0,002 | 0,002 | 0,002 | 0,056 | 0,054 |
| 1,1-Dichlorethan | 0,004 | 0,007 | 0,005 | 0,005 | 0,005 | 0,005 | 0,008 | 0,007 |
| $CCl_4$ | 0,091 | 0,099 | 0,127 | 0,125 | 0,078 | 0,086 | 0,112 | 0,132 |
| 1,2-Dichlor-ethylen-cis | 0,008 | 0,008 | 0,010 | 0,011 | 0,008 | 0,006 | 0,103 | 0,098 |
| $CHCl_3$ | 0,107 | 0,134 | 0,121 | 0,122 | 0,128 | 0,117 | 0,124 | 0,134 |
| 1,1,2-Trichlor-ethylen | 0,005 | 0,005 | 0,004 | 0,004 | 0,002 | 0,003 | 0,020 | 0,018 |
| 1,2-Dichlorethan | 99,119 | 99,065 | 98,969 | 98,960 | 99,225 | 99,201 | 97,053 | 96,954 |
| Tetrachlorethylen | 0,018 | 0,018 | 0,018 | 0,019 | 0,014 | <0,010 | 0,035 | 0,043 |
| 1,1,2-Trichlor-ethan | 0,397 | 0,394 | 0,498 | 0,496 | 0,307 | 0,335 | 1,386 | 1,669 |
| Ethylenchlorhydrin | 0,014 | 0,013 | 0,013 | 0,013 | 0,008 | 0,009 | 0,075 | 0,088 |
| Tetrachlorethan | 0,021 | 0,020 | 0,025 | 0,026 | 0,015 | 0,023 | 0,532 | 0,487 |

## Patentansprüche

1. Verfahren zur Herstellung von 1,2-Dichlorethan, wobei in mindestens einem Reaktionsraum Ethylen mit Chlor, gegebenenfalls in Gegenwart eines Katalysators, bei 20 bis 150°C und in mindestens einem weiteren Reaktionsraum Ethylen mit Chlorwasserstoff und Sauerstoff in Gegenwart eines Katalysators sowie gegebenenfalls unter den Reaktionsbedingungen inerter Gase, bei 180 bis 300°C umgesetzt wird, die gebildeten Reaktionsprodukte aus den Reaktionsräumen abgeführt werden und daraus die Hauptmenge des gebildeten 1,2-Dichlorethans abgeschieden wird, dadurch gekennzeichnet, daß das nach Abscheidung des 1,2-Dichlorethans aus den Reaktionsprodukten der Umsetzung des Ethylens mit Chlor übrigbleibende Gasgemisch gegebenenfalls unter Zuführung weiterer Gasströme in den Reaktionsraum eingeführt wird, in dem Ethylen mit Chlorwasserstoff und Sauerstoff umgesetzt wird.

2. Verfahren nach Anspruch 1, dadurch gekennzeichnet, daß dem Gasgemisch aus der Umsetzung von Ethylen mit Chlor noch Abgase zugemischt werden, die aus Destillationsapparaten zur Reinigung von 1,2-Dichlorethan und/oder aus Lagergefäßen für 1,2-Dichlorethan stammen, welche im Anschluß an die unvollständige thermische Spaltung von 1,2-Dichlorethan zur Gewinnung von Vinylchlorid nach Abtrennung des Vinylchlorids benutzt werden.

3. Verfahren nach einem der Ansprüche 1 oder 2, dadurch gekennzeichnet, daß das Gasgemisch aus der Umsetzung von Ethylen mit Chlor vor Einspeisung in den Reaktionsraum, in dem die Umsetzung von Ethylen mit Chlorwasserstoff und Sauerstoff stattfindet, auf 100 bis 200°C erwärmt wird.

4. Verfahren nach einem der Ansprüche 1 bis 3, dadurch gekennzeichnet, daß das Gasgemisch aus einer Umsetzung von Ethylen mit Chlor stammt, die in Gegenwart von flüssigem 1,2-Dichlorethan und einem Katalysator bei 20 bis 95°C durchgeführt worden ist.

5. Verfahren nach einem der Ansprüche 1 bis 4, dadurch gekennzeichnet, daß das Gasgemisch aus der Umsetzung von Ethylen mit Chlor auf seinem Gehalt an Chlorwasserstoff und Ethylen analysiert wird und das Analysenergebnis zur automatischen Steuerung der Einspeisung zusätzlicher Mengen von mindestens einem der Gase Chlorwasserstoff, Luft oder Ethylen in den Reaktionsraum benutzt wird, in dem Ethylen mit Chlorwasserstoff und Sauerstoff umgesetzt wird.

## Claims

1. A process for the production of 1,2-dichloroethane, in which ethylene is reacted with chlorine at 20 to 150°C in at least one reaction chamber, if appropriate in the presence of a catalyst, and ethylene is reacted with hydrogen chloride and oxygen or inert gases containing oxygen at 180 to 300°C in at least one further reaction chamber, in the presence of a catalyst, the reaction products which have been formed are removed from the reaction chambers and the bulk of the 1,2-dichloroethane formed is separated from them, characterized by introducing the mixture of gases remaining after separating the 1,2-dichloroethane from the reaction products of the reaction of ethylene with chlorine, optionally with addition of further gas strams, into the reaction chamber in which ethylene is reacted with hydrogen chloride and oxygen.

2. A process as claimed in claim 1, characterised in that exit gases originating from distillation equipment for the purification of 1,2-dichloroethane and/or from storage vessels for 1,2-dichloroethane which are used subsequently to the incomplete thermal cracking of 1,2-dichloroethane in order to obtain vinyl chloride, after the removal of the vinyl chloride, are mixed into the mixture of gases formed from the reaction of ethylene with chlorine.

3. A process as claimed in either of claims 1 or 2, characterized in that the mixture of gases formed from the reaction of ethylene with chlorine is warmed to 100 to 200°C before being fed into the reaction chamber in which the reaction of ethylene with hydrogen chloride and oxygen takes place.

4. A process as claimed in either of claims 1 to 3, characterized in that the mixture of gases originates from a reaction of ethylene with chlorine which has been carried out at 20 to 95°C in the presence of liquid 1,2-dichloroethane and a catalyst.

5. A process as claimed in either of claims 1 to 4, characterized in that the mixture of gases formed from the reaction of ethylene with chlorine is analysed to determine its content of hydrogen chloride and ethylene and the result of the analysis is used for the automatic control of the introduction of additional quantities of at least one of the gases hydrogen chloride, air or ethylene into the reaction chamber in which ethylene is reacted with hydrogen chloride and oxygen.

## Revendications

1. Procédé de préparation du dichloro-1,2 éthane dans lequel on fait réagir dans au moins un espace de réaction l'éthylène avec le chlore, éventuellement en présence d'un catalyseur, à 20 à 150°C et dans au moins un autre espace de réaction l'éthylène avec la chlorure d'hydrogène et l'oxygène en présence d'un catalyseur et éventuellement de gaz inertes dans les conditions de réaction, à 180 à 300°C, on enlève des espaces de réaction les produits de réaction et l'on en sépare la majeure partie du dichloro-1,2 éthane formé, procédé caractérisé en ce que, après la séparation du dichloro-1,2 éthane des produits de réaction de l'éthylène avec le chlore, on introduit le mélange gazeux résiduel, éventuellement avec acheminement d'autres courants gazeux, dans l'espace de réaction dans lequel on fait réagir l'éthylène avec le chlorure d'hydrogène et l'oxygène.

2. Procédé selon la revendication 1, caractérisé en ce qu'on ajoute encore au mélange gazeux provenant de la réaction de l'éthylène avec le chlore des gaz qui proviennent des appareils de distillation pour la purification du dichloro-1,2 éthane et/ou de récipients de stockage du dichloro-1,2 éthane et que l'on utilise après la coupure thermique incomplète du dichloro-1,2 éthane pour l'obtention du chlorure de vinyle, après séparation de ce chlorure de vinyle.

3. Procédé selon l'une des revendications 1 ou 2, caractérisé en ce qu'on chauffe jusqu'à 100 à 200°C le mélange gazeux provenant de la réaction de l'éthylène avec le chlore, avant de l'introduire dans l'espace de réaction dans lequel se produit la réaction de l'éthylène avec le chlorure d'hydrogène et l'oxygène.

4. Procédé selon l'une des revendications 1 à 3, caractérisé en ce que le mélange gazeux provient d'une réaction de l'éthylène avec le chlore qui a été effectuée à 20 à 95°C en présence de dichloro-1,2 éthane liquide et d'un catalyseur.

5. Procédé selon l'une des revendications 1 à 4, caractérisé en ce qu'on analyse, pour en déterminer la teneur en chlorure d'hydrogène et en éthylène, le mélange gazeux provenant de la réaction de l'éthylène avec le chlore et en ce qu'on utilise le résultat de l'analyse pour la commande automatique de l'introduction de quantités supplémentaires d'au moins l'un des gaz chlorure d'hydrogène, air, ou éthylène dans l'espace de réaction dans lequel on fait régir l'éthylène avec le chlorure d'hydrogène et l'oxygène.